# EUROPEAN PATENT APPLICATION

(11) **EP 1 216 618 A1**
(43) Date of publication of application: **26.06.2002**
(21) Application number: 00204684.5
(22) Date of filing: 20.12.2000
(51) Int. Cl.: A23C 19/032, A23C 19/06, C12N 15/74

(54) **Cheese product and method for the preparation thereof**

(71) Applicant: CAMPINA MELKUNIE B.V., NL-5301 LB Zaltbommel (NL)
(72) Inventor: Bruinenberg, Paul Gerard, 5071 KE Udenhout (NL)
(74) Representative: Wittop Koning, Tom Hugo

(57) **Abstract**

Described is a method for the preparation of cheese and cheese based products comprising the step of adding bacteria of at least one recombinant cystathionine-β-lyase (CBL) overproducing bacterial strain to material from which the cheese product is formed and a cheese product, obtainable by the said method. The cheese product has an enhanced sulphur, sweet, brothy and fruity taste compared to a corresponding conventionally produced cheese product.

## Description

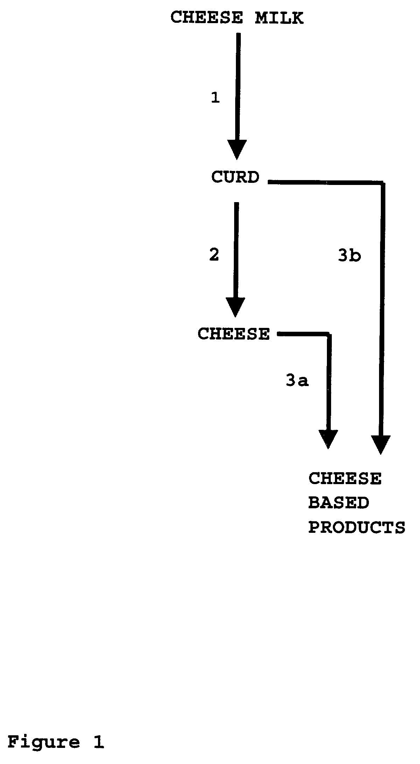

The present invention relates to a method for the preparation of a cheese product, having an improved taste and aromatic properties.
In the art, methods for the preparation of cheese products are generally known, and involve curding milk and ripening of the curd into cheese products using lactic acid bacteria. The main steps in a standard cheese making process are elucidated in figure 1. With "cheese products" products are meant that are obtainable from curd that has been subjected to a ripening process. The term "cheese-products" therefore encompasses e.g. curd cheese, common cheese, such as cottage cheese, Gouda, Parmesan, Cheddar, etc., and varieties thereof, as well as cheese based products, such as processed cheese products, cheese analogues, cheese paste and enzyme modified cheese. The above mentioned terms are known in the art; for an overview of the above, see the textbook "Cheese: chemistry, physics and microbiology", 2^{nd} edition, P.F. Fox, ed. Chapman and Hall, London, Great-Britain, 1993.

Lactic acid bacteria, such as Lactococci, Lactobacilli, Leucostonocs and *Streptococcus thermophilus* play an important role in the different process steps of cheese preparation, such as in the curding process. In the process, milk proteins are digested into peptides, that subsequently are cleaved into individual amino acids. These amino acids already confer a basic taste to the cheese. In addition, said amino acids can be converted into more or less volatile compounds, some of which comprising sulphur, that confer the typical cheese taste during the ripening process.

The cheese ripening process is slow, as the required enzymes are released from the lactic acid bacteria after permeabilisation or lysis thereof. Therefore, the ripening material, leading to the cheese product once the ripening procedure is completed, is to be stored for a considerable time at controlled conditions, which is an important cost factor.

Therefore, attempts have been made to accelerate the ripening process by a.g. adding enzyme preparations enhancing the digestion of proteins and cleavage of the peptides, and by using lactic acid bacteria having enhanced permeabilisation or lysis characteristics. Such bacteria become permeable or lyse, respectively, within a shorter period than the conventional lactic acid bacteria, so that the ripening process is accelerated.

The present invention provides an improved method for the preparation of a cheese product, wherein the time needed for the ripening process can be further reduced, and wherein the cheese products have enhanced and/or altered taste and aromatic properties. The method is characterised in that it comprises the step of adding at least one recombinant cystathionine-β-lyase (CBL) overproducing bacterial strain to the material from which the cheese product is formed.

With "altered taste", mainly a so-called sulphur and thermophilic taste (sweet, brothy and fruity) is meant. According to the invention, this sulphur and thermophilic taste can be enhanced or provided in cheese products. The method according to the invention therefore also relates to a method for the preparation of a cheese product having improved and/or altered taste comprising the step of adding bacteria of at least one recombinant cystathionine-β-lyase (CBL) overproducing bacterial strain to material from which the cheese product is formed.

"Material from which the cheese product is formed" is defined as any material used in the cheese preparation process, which itself, or a product converted therefrom, is present in the resulting cheese product. Examples of such materials is the starting material milk and any intermediate product such as curd or cheese in the process of being ripened, such as e.g. curd cheese or cheddar cheese, although curd cheese or cheddar cheese may as well be the final cheese product to be produced by the method of the present invention. The ripened cheese may however also be regarded as "material from which the cheese product is formed", e.g. in case processed cheese products are to be prepared by adding bacteria to the ripened cheese, e.g. after a heating step. According to the invention, the bacteria may therefore be added to the starting material milk, or to any intermediate products such as curd, or to the cheese after ripening. The bacteria may also be added at different time points in the cheese preparation process, e.g. to the starting material and to an intermediate product.

The enzyme cystathionine-β-lyase (CBL) catalyses the conversion of the amino acid methionine in methane thiol, which is a direct precursor of the volatile aromatic compounds in many cheese types, such as Cheddar and Gouda. Said aromatic compounds confer the characteristic taste to the cheese. CBL is produced by the conventional lactic acid bacteria in relatively low amounts (0,014 U/mg protein; see for the definition of CBL units Alting et al. (1995) Appl. Env. Microbiol. vol. 61, pp 4037-4042). The present inventors have succeeded to obtain a cheese product having excellent taste and aromatic properties by adding during the preparation process, bacteria of at least one recombinant CBL overproducing bacterial strain. "Recombinant CBL overproducing bacterial strain" is defined as a bacterial strain being genetically engineered such that it produces more CBL activity than the parent strain in which the genetic engineering has been carried out. The bacterial strain may as well be a genetically engineered recombinant strain, however, it is to be understood that said genetic engineering in the bacterial strain has not led to an increase of production of CBL activity. Such a genetic engineered parent strain may e.g. be genetically engineered to obtain other characteristics than overproduction of CBL activity. "CBL activity" is defined as units thiol generated/mg protein using cystathionine as a substrate in the method described by J.R. Uren (Methods Enzymology, 1987, vol. 143, pp. 483-486).

The recombinant CBL overproducing bacterial strain may comprise its original CBL coding sequence, the *metC* gene, being operably linked to genetic elements that improve the expressing of the said endogenous *metC* gene. Said elements are known in the art and comprise e.g. promoter and of/or enhanced sequences. The overproducing strain may however comprise CBL encoding nucleic acid sequences, not present in the parent strain. Said additional encoding sequences may be derived from the same bacterial species, but may also be of heterologous origin, such as from Lactobacilli species and *Streptococcus thermophilus.*

By adding a recombinant CBL overproducing bacterial strain in the cheese preparation process, an increased amount of CBL activity will become available in the material from which the cheese product is formed, therewith resulting in a cheese product of improved taste and having altered aromatic properties. Further, the ripening process may be accelerated.

In an attractive embodiment of the invention, at least one non-recombinant CBL producing bacterial strain is added to the material from which the cheese product is formed. "Non-recombinant CBL producing bacterial strain" is defined as any bacterial strain that is free of any recombinant DNA that would lead to enhanced production of CBL activity. Such a strain may however be a recombinant, wherein the genetically engineered sequences do not contribute to enhancement of the CBL production of the said bacteria. An example of such non-recombinant CBL producing bacterial strains are the conventional lactic acid bacteria used in the art in the cheese preparation process. By combining both a recombinant CBL overproducing strain and a non-recombinant "normal" CBL producing strain, the production of CBL activity during the cheese preparation process, can be adjusted. Further "helper" strains can be used, that themselves do not show improved production of CBL activity, or may not even be involved in the common cheese preparation process, but may produce agents required for improving the growth of and/or the induction of CBL production or release in the recombinant CBL overproducing bacterial strain.

Preferably, the amount of added non-recombinant CBL producing bacteria is at most equal to the amount of added recombinant CBL overproducing bacteria. When the amount of recombinant bacteria is less than that of the non-recombinant bacteria, the improvement in taste and aromatic characteristics can not always be guaranteed, e.g. due to insufficient activity and/or lack of permeability or lysis of the recombinant strain (see below). The dosage of non-recombinant cells to the cheese milk in a standard cheese preparation process for e.g. Gouda is in such a case for example about 0,6% (v/v) of a full grown culture. The amount of recombinant cells is at least equal to 0,6% (v/v).

Preferably, and most notably in the curding step, the amount of added non-recombinant CBL-producing bacteria: the amount of added recombinant CBL overproducing bacteria is 1:1,5-5, preferably 1:1,8-3,0, most preferably 1:1,9-2,5. However, it is also possible to use a relative higher amount of recombinant CBL overproducing bacteria. In particular, in the preparation process of processed cheese from curd or from cheese (the ripened curd), the said ratio may be substantially higher; the process can even, and advantageously, be carried out by the addition of recombinant CBL overproducing bacteria in the absence of non-recombinant CBL-producing bacteria. In the case of the production of a processed cheese product from curd or cheese according to the present invention, the process for the preparation of the said curd or cheese may however involve the addition of common non-recombinant CBL producing bacteria, optionally combined with recombinant CBL overproducing bacteria.

In the above, when the preparation process involves both the addition of recombinant CBL overproducing bacteria and of non-recombinant CBL producing bacteria, the indicated amounts and ratios therebetween are to be regarded as the respective amounts/ratios of added bacteria in the same step of the cheese preparation process. Addition of the non-recombinant CBL producing be simultaneous with the recombinant CBL overproducing bacteria, e.g. as a mixture; however, both non- recombinant and recombinant bacterial strains may also be subsequently within in a short time period within the same stage of the preparation process, where the order of addition may not be critical and can be determined by the skilled practitioner. It is however also possible to add bacteria in more stages during the cheese preparation process; in that case, in each stage both recombinant CBL overproducing bacteria and non-recombinant CBL producing bacteria can be added. As is outlined above, it is even possible to add recombinant CBL overproducing bacteria without addition of non-recombinant CBL producing bacteria in one or more stages of the cheese preparation process.

The added recombinant CBL overproducing bacteria preferably belong to a lactic acid bacteria strain but may also be of another species, such as e.g. a *Brevibacterium* strain, or of a bacterial strain that is not involved in the common cheese preparation process. However, it is important that such a bacterial strain is non-pathogenic and food-grade, having the co-called "GRAS"(generally recognised as safe)-status. Preferably however, all CBL producing bacterial strains, i.e. including the recombinant CBL overproducing bacterial strain(s), are bacteria, involved in the common cheese preparation process, and are most preferably lactic acid bacteria.

The expression of the CBL from the recombinant CBL overproducing bacterial strain is preferably induced. Thus, the recombinant strain can be made to overproduce CBL activity by controlling the conditions wherein the CBL expression is induced. In the art, inducible expression systems are well known; e.g., for Lactococci, the *rlt* system is described in Nauta et al, 1997, Nature Biotechnology, vol. 15, pp. 980-983 and may be used according to the invention. Preferably, the CBL expression from the recombinant CBL overproducing bacterial strain is inducible by nisin. An inducible heterologous gene expression system, inducible by nisin is known from EP 0712935, under the trade name NICE™. In summary, the heterologous gene, in the present case a gene encoding CBL activity, is operably linked to the nisin promoter. The expression under control of the nisin promoter increases with the amount of nisin, available to the bacteria. In a special embodiment, an additional inducer producing bacterial strain, such as in this case a nisin producing strain, such as *L. lactis* subspecies. lactis var. *diacetylactis* TC 17-5 (commercial NIZOSTAR culture of CSK food enrichment, Leeuwarden, The Netherlands), is added in the cheese preparation process, so that sufficient inducer is present for induction of the CBL encoding sequence on the recombinant CBL overproducing bacterial strain.

In an attractive embodiment, the expression of CBL from the recombinant CBL overproducing strain is induced before adding of the said strain to the material from which the cheese product is formed. When the recombinant strain is already induced before adding in the cheese preparation process, the CBL gene expression is already switched on and the recombinant bacteria are able to overproduce CBL activity directly after adding thereof in the process, therewith avoiding the phase of low CBL production, during induction process. The recombinant bacteria may already overproduce CBL in the preceding injection step, so that in combination of already induced recombinant bacteria as well as already produced CBL is simultaneously added in the cheese preparation process.

Preferably, the CBL activity produced per recombinant CBL overproducing bacterial cell is at least ten times, preferably at least twenty times, the CBL activity produced per non-recombinant CBL producing bacterial cell. Less enzyme activity is not preferred, since in that case flavour compound production is likely below the threshold level for organoleptical evaluation.

It is very advantageous when the material from which the cheese is formed comprises an agent, enhancing the release of CBL from at least the recombinant CBL overproducing bacteria in the material from which the cheese product is formed. Such an agent may induce the permeability of the bacterial membrane, so that the CBL molecules can "leak out" of the cell, or may enhance lysis of the recombinant CBL overproducing bacteria. By the presence of such a lytic agent, the recombinant bacteria will be more susceptible to lysis, leading to a better availability of CBL in the material, therewith enhancing the cheese ripening process which may result into improvement of cheese taste and of decreasing the period needed for cheese ripening and therewith storage time. The release enhancing agent may be added during the cheese preparation process in the form of a compound formulation or may be produced by bacteria or other micro-organisms that may be added during the cheese preparation process. It is also possible that e.g. the recombinant CBL-overproducing bacteria themselves are capable to produce a lytic agent, leading to lysis of the cells. Such endogenous production of a lysis enhancing agent may also be provided by an inducible expression system or may be produced through constitutive expression of a gene encoding such a lysis enhancing agent. In a preferred embodiment however, the CBL release enhancing agent, preferably a lytic agent, is induced by an additional bacterial strain that is added to the material from which the cheese product is formed. In the said strain the expression of the gene encoding the lysis enhancing agent may also be constitutively or inducibly be expressed.

Preferably, the recombinant CBL overproducing strain is *Lactococcus lactis* NZ3900, wherein a CBL-encoding nucleic sequence is operably linked to a high expression promoter, functional in the said bacteria. NZ3900 is chosen as a host since it carries in the *pepN* locus the *nisR* and *nisK* genes, required for signal transduction of nisin (Kuipers e al, 1993. European Journal Biochemistry, vol. 216, pp. 27299-27304). In addition, NZ3900 carries a deletion in the chromosomal *lacF*-gene and therefore cannot grow on lactose (de Ruyter et al., 1996, J. Bacteriol., vol 178, pp. 3434-3439). Transformation of NZ3900 with plasmid pNZ8143 harbouring the food-grade *lacF* marker restores the ability to grow on lactose as a sole carbon source (Platteeuw et al, 1996, Applied and Environmental Microbiology, vol. 62, pp. 1008-1013). The parent strain of *Lactobacillus lactis* NZ3900, NZ9000, is described in N. Fernandez et al, Applied Microbiology 66:42-48(2000). Extracts of *L. lactis* cells of strain NZ9000, harbouring pNZ8137, which overproduce CBL, were shown to form larger amounts of sulphur compounds from methionine than the wild-type strain in an *in vitro* assay (Fernandez et al, 2000, Applied Microbiology 66:42-48).

Preferably, the recombinant CBL overproducing bacterial strain comprises one or more copies of a plasmid chosen from pNZ8140 (deposited at the Centraal Bureau voor Schimmelcultures (CBS), P.O. Box 85167, Utrecht, The Netherlands on december 13, 2000 according to the requirements of the Budapest Treaty under accession number CBS 109210) and pNZ8143(deposited at the CBS on december 13, 2000 according to the requirements of the Budapest Treaty under accession number CBS 109211). Plasmid pNZ8143 is a derivative of plasmid pNZ8137 (Fernandez et al, 2000), in which the Cm^{R}-gene is replaced by the lacF gene as a food-grade selectable marker. Furthermore, a stretch of 60 bp *Bacillus subtilis* DNA is removed from the original plasmid. The plasmid further carries the pHS71 replicon and the *L. lactis* B78 *metC* gene, encoding cystathionine-β-lyase, under the control of the nisin promoter. The resulting plasmid pNZ8143 consists only of L. lactis DNA and can therefore be regarded as a food-grade vector (see Lindgren (1999), Int. Dairy J. vol. 9 pp. 37-41; see also example 2)

In a preferred embodiment, the invention also relates to the novel plasmids pNZ8140 and pNZ8143, which are extremely useful for the preparation of recombinant bacteria, capable of overproducing CBL.

The invention will be further illustrated by the following examples and figures, wherein
Figure 1 shows a schematic diagram of a common cheese preparation process,
Figure 2 shows a scheme of the cloning strategy for the construction of pNZ8140, and
Figure 3 shows plasmid maps of pNZ8140, pNZ8141, pNZ8142 and pNZ8143.
In the scheme of figure 1, the common cheese preparation process is illustrated. Starting material is cheese milk, which can be any kind of milk, such as cow milk, goat milk, sheep milk, etcetera.

In a first step, also indicated as curding step, and indicated with arrow 1, bacteria and/or enzymes (amount and species depends on the kind of cheese to be obtained) are added to the cheese milk, resulting to curd. During step 1, recombinant CBL overproducing bacteria may be added to the milk, with or without the addition of non-recombinant CBL producing bacteria. The non-recombinant CBL producing bacteria are usually commonly used lactic acid bacteria.

The second step (arrow 2) is the ripening stage, wherein the curd is ripened to cheese by storing the curd at the proper conditions, depending on the kind of cheese to be produced. In the ripening stage, usually no bacteria are added. The invention however also encompasses variants of the cheese preparation process, wherein CBL producing bacteria are added to the curd during the ripening stage.

From the cheese, but also from the curd, cheese based products can be produced by a third processing step (arrows 3a and 3b, respectively), comprising heating the cheese or curd, followed by the addition of bacteria and/or enzymes. This third processing step is often done in countries where processed or cheese analogues are popular.

According to the invention, in each of the steps, the recombinant CBL overproducing bacteria can be added, with or without addition in the said same step of non recombinant CBL producing bacteria. When, in case of the preparation of cheese based products, bacteria are added in two different stages (stages 1 and 3), in one or each of the said stages recombinant CBL overproducing bacteria can be added, with or without addition of non-recombinant CBL producing bacteria; e.g., in step 1 both recombinant CBL overproducing bacteria and non-recombinant CBL producing bacteria can be added, whereas, later in step 3 of the process, only recombinant or only non-recombinant bacteria can be added. However, in a special embodiment of the invention, in step 3 recombinant CBL overproducing bacteria are added, without the addition of non-recombinant CBL producing bacteria.

### Example 1

### Food-grade overproduction of CBL using NICE™-system

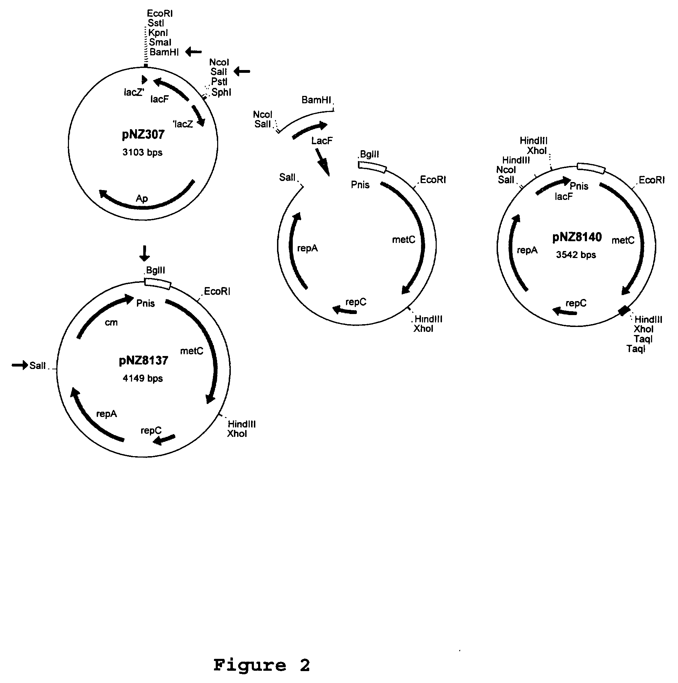

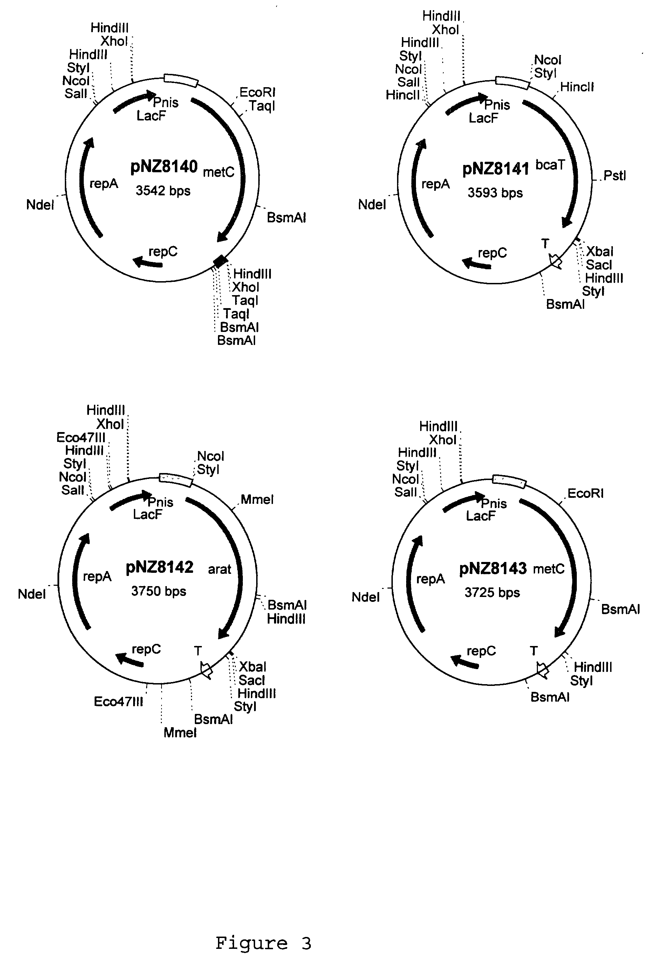

To demonstrate that cystathionine β-lyase (CBL) can be overproduced in a food-grade manner in *L. lactis* using the NICE™ system, the below mentioned procedure for construction of plasmids was followed. In this study the methionine-converting enzyme CBL of *L. lactis* strain NIZO B78 was expressed with the NICE™ system. For this purpose a food-grade plasmid was constructed that make use of the food-grade selection marker *lacF*. Plasmid pNZ8137 contains the *metC* gene, coding for cystathionine β-lyase, of *L. lactis* strain B78 cloned under control of the *nisA* promoter of pNZ8037 (M. Fernández et al., 2000 Appl. Environ. Microbiol., vol. 66, 42-48).
A food-grade derivative, pNZ8140, was constructed by replacement of the *cat* gene encoding chloramphenicol resistance by the food-grade marker *lacF* (Figure 2). Expression of the promoter-less *lacF* gene is obtained by the readthrough from the *repA* gene. Careful analysis of the complete sequence of plasmid pNZ8140 showed that it contains a stretch of 60 bp originating from *Bacillus subtilis*. Although this is not supposed to harm the self-cloning status of the plasmid, it was decided to construct a new cloning vector without the *B. subtilis* DNA-fragment that, consequently, only consists of *L. lactis* DNA. Therefore, a fragment containing this 60-bp fragment was deleted from the NICE™ plasmid pNZ8048 resulting in plasmid pNZ8148. The *metC* gene was cloned from plasmid pNZ8137 into pNZ8148 yielding pNZ8148-*metC*. Subsequently, the *cat* gene was replaced by *lacF* resulting in the food-grade plasmid pNZ8143 (Figure 3; therein, terminators are indicated by an open arrow. The *B. subtilis* sequence in pNZ8140 is indicated as a black box).
Plasmid pNZ8143 was transformed into strain *L.lactis* NZ3900 (de Ruyter et al., 1996, J. Bacteriol. vol. 178, pp. 3434-3439). NZ3900 is chosen as a host since it carries in the *pepN* locus the *nisR* and *nisK* genes, required for signal transduction of nisin (Kuipers et al.,1993, Eur. J. Biochem., vol, 216, 27299-27304). NZ3900 also carries a deletion in the chromosomal *lacF* gene and therefore cannot grow on lactose (de Ruyter et al., 1996, J. Bacteriol. vol. 178, pp. 3434-3439). Transformation of NZ3900 with plasmid pNZ8143 harbouring the food-grade lacF marker restores the ability to grow on lactose as a sole carbon source (Platteeuw et al., 1996, Appl. Environ. Microbiol, vol. 62, 1008-1013).
For CBL overproduction with the NICE^{TM} system, *L. lactis* NZ3900 harboring pNZ8143 was cultured in M17 medium supplemented with 0.5% lactose to an optical density at 600 nm (OD₆₀₀) between 0.5 and 0.7, induced with 0, 0.1, or 1.0 ng nisin A per ml and incubated for another 2 hours at 30°C. Cell-free extracts were prepared and CLB activity towards cystathionine was measured by determination of free thiol group formation with DTNB as described by Uren (Methods in Ezymology, 1987, vol. 143, pp. 483-486) using 13200 ml/mmol.cm as the extinction coefficient to calculate the results.
The cystathionine lyase (CBL) activity was determined for the cell-free extracts of the cultures overproducing CBL (Table 1). Induction with 1 ng nisin/ml resulted in a 23-fold increase in CBL activity compared to the uninduced culture. As a control *L. lactis* NZ9000 harbouring pNZ8137 was used (M. Fernández et al., 2000. Appl. Environ. Microbiol., vol. 66, 42-48). The specific activity of this strain was 2.0 for an uninduced culture and 273 for the culture induced with 1.0 ng nisin per ml. The specific CLB activity of *L. lactis* NIZO B78 grown in milk is 14 nmol per mg protein per minute (A. C. Alting et al.,1995. Appl. Environ. Microbiol., vol. 61, 4037-4042). The CLB activity of strain NZ9000 is more than 10-fold lower than that of NZ3900 harbouring pNZ8143, while strain B78 has an intermediate activity. The difference in CLB activity between strain NZ3900 and NZ9000 may be due to the fact that these host strains are not identical (the difference is that NZ3900 contains a chromosomal integration of the lactococcal lactose operon (with *lacF* deleted)). Nevertheless, these results show that a 50-fold overproduction of CLB specific activity is achieved in fully induced cells of NZ3900 carrying pNZ8143 when compared with the natural wild-type strain *L*. *lactis* NIZO B78.

**Table 1.**

| Specific and relative CLB activities of *L. lactis* NZ3900 harbouring pNZ8143. | | |
|---|---|---|
| Concentration of nisin A (ng/ml) | Specific Activity (nmol of mercaptide formed per mg protein per min) | Relative Activity |
| 0.0 | 30 | 1.0 |
| 0.1 | 46 | 1.5 |
| 1.0 | 690 | 23.0 |

### Example 2

### The effect of CBL overproduction on flavour development in cheese paste.

Current legislation does not allow the cheese graders to taste a cheese paste with high inoculation levels of *L. lactis* NZ3900 harbouring pNZ8143, because this strain is still considered as a genetically modified microorganism. Therefore, to study the effect of CBL overproduction on flavour development in a cheese paste permeabilised cells were used. With this approach the enzymes are retained inside the cell, while the substrate molecules and flavour compounds can diffuse freely into or out of the cell. Therefore, flavour formation will not depend on active transport of substrate molecules into the cell and diffusion of the flavour compounds to the cheese paste.

Firstly, it was tested whether this method could be used for CBL, by comparison of CLB activity of cell-free extracts made from the same culture of *L. lactis* NZ3900 harboring pNZ8143 that were either permeabilised with various concentrations of butanol or untreated. Cells from a log phase-culture were divided in two batches. One was incubated with 5% butanol for permeabilisation. The butanol was removed by washing 5 times with a buffer and the cells were used to make cell-free extracts. The other batch was not treated with butanol and immediately used to make cell-free extracts. Treatment with 5% butanol did not effect the CLB activity (Table 2), while it reduced the amount of living cells approximately 10⁹-fold compared to a reduction of 10-fold for 3% butanol (data not shown).

**Table 2.**

| Effect of permeabilisation on CLB activity. | | | | |
|---|---|---|---|---|
| | control | 3 % butanol | 4 % butanol | 5 % butanol |
| Relative CLB activity | 1 | 1.15 | 0.94 | 0.98 |
| CLB activity in cell-extracts of *L. lactis* cells (control), cells permeabilised with 3, 4 and 5 % butanol relative to that of non permeabilised cells. | | | | |

In this experiment the CLB activity of the induced cells was approximately 12 times higher than that of the uninduced cells. The cheese pastes were inoculated in duplicate with water (blanc), ∼10⁹ cells/gram paste of *L. lactis* NZ3900 harbouring pNZ8143 precultured in LM17, or ∼10⁹ cells/gram paste of *L. lactis* NZ3900 harbouring pNZ8143 precultured in LM17 and induced with 2 ng nisin per ml culture. The cheese pastes were incubated at 13°C for 3 weeks after which one series was rated by 9 persons for aroma and taste and the other was used for sulphur analysis using HS-GC. In addition, a cheese paste was inoculated with ∼10⁹ cells/gram paste of *L. lactis* NZ3900 which were not treated with butanol. This latter cheese paste was only subjected to GC-analysis, because graders were not allowed to taste a cheese paste with untreated cells (see above).
After 3 weeks incubation, the cheese paste containing the induced cells was unanimously graded as having more Gouda flavour and was sometimes judged as more thermophilic (sweet and broth). In addition, this cheese paste harbored a sulphur-flavour note as judged by the graders (Table 3). In contrast, the cheese with the uninduced cells was very much like the water-supplemented cheese paste and was only judged to be less flat. This result shows that overproduction of cystathionine β-lyase not only accelerates Gouda flavour development, but in addition produces an extra sulphur/thermophilic flavour in a cheese paste.

**Table 3.**

| Grading scores of cheese pastes after 3 weeks incubation at 13°C. | | |
|---|---|---|
| | Gouda flavour | Sulphur/thermophilic- flavour intensity |
| blank, no cells added | 6.0 | 0 |
| non-induced cells (permeabilised) | 6.2 | 0 |
| nisin-induced cells (permeabilised) | 6.6 | 0.3 |
| Scales: aroma/taste Gouda from 4-8, and thermophilic/sulphur from 0-4. | | |

HS-GC analysis of the cheese pastes (Table 4) confirmed the findings of the grading. The cheese paste incubated with induced cells showed a significant increase in amount of volatile sulphur compounds methanethiol (0.5 -fold), dimethylsulfide (3-fold) and dimethyldisulphide (3-fold) when compared with uninduced cells. In contrast, the amount of carbondisulfide remained constant because this compound is not produced by the action of cystathionine β-lyase.

Furthermore, treatment with butanol of induced cells increases the amount of above mentioned volatiles in the cheese paste with 30-50 %, indicating that a permeabilised cell membrane enhances sulphur-compound formation produced by the enzyme cystathionine β-lyase in the cheese paste.

**Table 4.**

| Sulphur compounds detected in cheese pastes. | | | | |
|---|---|---|---|---|
| cells | methanethiol (ppb) | dimethylsulfide (ppb) | dimethyldisulfide (ppb) | carbonsulfide (ppb) |
| permeabilised uninduced | 29500 | 12000 | 7500 | 5000 |
| permeabilised induced | 44500 | 50500 | 30500 | 6000 |
| non permeabilised induced | 29000 | 26000 | 20500 | 5500 |
| Sulphur compounds detected in cheese pastes incubated with permeabilised *L. lactis* cells overproducing CBL (induced), permeabilised cells (uninduced) and control (non-permeabilised, induced) cells. Methanethiol, dimethylsulfide, and dimethyldisulfide, are produced by methionine lyase activity, while carbondisulfides are not. | | | | |

### Example 3

### The effect of CBL overproduction on flavour development in Gouda-type cheese.

To establish the effect of the recombinant CBL-overproducing strain on flavour formation in cheese, Gouda cheese trials were performed. Gouda cheese was prepared from 200 L portions of pasteurized milk (10 sec., 74 °C) according to standard Gouda cheesemaking procedures (Walstra, P,, Noomen, A. & Geurts, T.J. (1987) Dutch-type varieties. In P.F. Fox, Cheese: chemistry, physics and microbiology (pp. 45-92). Londen: Elsevier). The traditional Bos cheese starter culture was cultivated in pasteurised skimmed milk for 16 hours at 18 °C. Culture NZ3900 harboring pNZ8143 (CBL overproducer) was grown in LM17-broth and induced with nisin as indicated in example 1. The nisin-producer TC17-5 (commercial CSK culture, Leeuwarden, The Netherlands) was added to the cheesemilk by direct inoculation from the concentrate culture. The used culture dosages in cheese trials are indicated in Table 5. Since the dosage of the nisin-producing culture to the cheese milk is very low, nisin concentrations in cheese are subinhibitory and no effect was observed on the acidification rate during cheese making. After brining the cheeses were ripened at indicated periods at 13 °C en 85 % relative air moisture.

**Table 5.**

| Culture dosage in percentage (v/v). | | | |
|---|---|---|---|
| trial | BOS (%) | NZ3900 + pNZ8143 (%) | TC17-5 (%) |
| 1 | 0.6 | - | 0.005 |
| 2 | 0.6 | 1 (non-induced) | 0.005 |
| 3 | 0.6 | 1 (induced) | 0.005 |
| 4 | 0.6 | 1 (induced) | - |

The cheeses were graded by a trained panel consisting of 8 persons at 6, 13 and 16 weeks of ripening. At 6 weeks of ripening the graders did not observe any organoleptical differences between the cheeses of various cheese trials. The gradings results at 13 and 16 weeks of ripening are shown in Table 6.

**Table 6.**

| Grading results of Gouda cheese prepared with various culture dosages (see Table 5). | | | | | | | | |
|---|---|---|---|---|---|---|---|---|
| | 13 weeks ripening | | | | 16 weeks ripening | | | |
| | 1 | 2 | 3 | 4 | 1 | 2 | 3 | 4 |
| aroma/ taste | 6.2 ± | 6.2 ± | 6.5 ± | 6.2 ± | 6.3 ± | 6.4 ± | 6.3 ± | 6.5 ± |
| | 0.4 | ± 0.5 | ± 0.4 | ± 0.3 | 0.3 | 0.3 | 0.3 | 0.5 |
| Thermophilic/ sulphur | 0.2 ± | 0.2 ± | 0.3 ± | 0.5 ± | 0.2 ± | 0.7 ± | 0.8 ± | 0.9 ± |
| | ± 0.2 | ± 0.3 | ± 0.4 | ±0.4 | 0.3 | 0.2 | 0.3 | 0.3 |
| Scales: aroma/taste from 4-8, and thermophilic/sulphur from 0-4. | | | | | | | | |

After 13 weeks ripening a trace of thermophilic/sulphur flavour is observed in cheeses prepared with NZ3900 harboring pNZ8143 (trial 3 and 4). However, since this extra flavour was not observed by all graders in these cheeses, the statistical relevance, as indicated by the standard deviation in Table 6, is relatively low. The overall score for aroma and taste was similar for all cheeses.
In contrast, at 16 weeks ripening the graders observed a clear additional flavour, specified as thermophilic and sulphur, in particular in cheeses prepared in trials 3 and 4. Since no significant flavour differences were observed between cheeses 3 and 4, this indicates that in the case cheese contain preinduced recombinant cells, the addition of an additional nisin-producing strain TC17-5 has no significant effect on flavour development. However, in cheese prepared with a non-induced recombinant strain, the addition of the nisin-producing strain TC17-5 (trial 2) causes also an additional thermophilic/sulphur-aroma to the cheeses, but less in intensity in comparison with cheese of trials 3 and 4 (Table 6). Apparently, in trial 2 the inducer strain TC17-5 produces such amount of nisin in cheese that the NICE™-system is induced suboptimally and less CBL is overproduced in recombinant cells in comparison with fully pre-induced cells as applied in trials 3 and 4. Subsequently, this relatively reduced CLB activity in trial 2 results in less thermophilic/sulphur flavour development. In the patent EP 0712935 is described that the level of induction of the NICE™-system is nisin dosage dependent.
Finally, the grading results indicates that the overall scores for aroma and taste at 16 weeks ripening for all cheeses are similar.
In summary, these results show that addition of a recombinant strain overproducing CBL to the cheese milk together with a traditional Gouda cheese starter (Bos), produces an extra thermophilic and sulphur flavour to the Gouda cheese at 16 weeks of ripening. Thus the CBL overproducing strain is able to diversify Gouda cheese flavour development. The dosage of this recombinant strain in comparison with Bos-starter, is preferably at least equal.
The effects of recombinant CBL strain on flavour development were only clearly observed by the cheese graders after a relative long ripening period of 16 weeks. This is likely due to the fact that the recombinant strain NZ3900 carrying pNZ8143 lyses relatively poor in the cheese matrix, thereby resulting in suboptimal realisation of CLB activity towards its substrate methionine. This is confirmed by the cheese paste experiment which shows that permeabilisation of the cell membrane of strain NZ3900 carrying pNZ8143 enhances flavour development (example 2).
The effect of improved CBL release from the recombinant CBL overproducing strain on Gouda flavour development by lysis/permeabilisation was tested in a similar trial, and gave the expected results, in line with the above.

## Claims

1. Method for the preparation of a cheese product comprising the step of adding bacteria of at least one recombinant cystathionine-β-lyase (CBL) overproducing bacterial strain to material from which the cheese product is formed.

2. Method according to claim 1, further comprising the step of adding bacteria of at least one non-recombinant CBL-producing bacterial strain to the material from which the cheese product is formed.

3. Method according to claim 2, wherein the amount of added non-recombinant CBL-producing bacteria is at most equal to the amount of added recombinant CBL overproducing bacteria, the ratio of the amount of added non-recombinant CBL-producing bacteria: the amount of added recombinant CBL overproducing bacteria preferably being 1:1,5-5, more preferably 1:1,8-3,0, most preferably 1:1,9-2,5.

4. Method according to claim 2 or 3, wherein the CBL activity produced per added recombinant CBL overproducing bacterial cell is at least 10 times, preferably at least 20 times, the CBL activity produced per added non-recombinant CBL producing bacterial cell.

5. Method according to any of the preceding claims, at least one of the added CBL-producing bacterial strains being lactic acid bacteria, preferably all CBL-producing bacterial strains being lactic acid bacteria.

6. Method according to the preceding claims, wherein the expression of the CBL from the recombinant CBL-overproducing bacterial strain is inducable, preferably by nisin.

7. Method according to any of the preceding claims, wherein the material from which the cheese is formed comprises an agent enhancing the release of CBL from at least the recombinant CBL-overproducing bacteria in the material from which the cheese product is formed, the agent preferably enhancing the lysis of at least the recombinant CBL-overproducing bacteria, and the CBL release enhancing agent preferably being produced by a bacterial strain, added to the material from which the cheese product is formed.

8. Method according to any of the preceding claims, wherein the recombinant CBL-overproducing bacterial strain is Lactobacillus lactis NZ3900, comprising a plasmid, wherein a CBL-encoding nucleotide sequence is operably linked to a high-expression promoter, functional in the said bacteria, the plasmid preferably being chosen from pNZ8140 (CBS 109210) and pNZ8143 (CBS 109211).

9. Plasmid, chosen from the group, consisting of pNZ8140(CBS 109210) and pNZ8143(CBS 109211).

10. Cheese product, obtainable by the method according to any of the claims 1-8.
